# EUROPEAN PATENT APPLICATION

(11) **EP 3 967 320 A1**
(43) Date of publication of application: **16.03.2022**
(21) Application number: 20802512.2
(22) Date of filing: 06.05.2020
(51) Int. Cl.: A61K 36/88, A61K 36/185, A61K 9/48, A23L 29/206, A61P 25/24

(54) **FOOD SUPPLEMENT FOR IMPROVING THE MOOD OF AN INDIVIDUAL**

(30) Priority: 08.05.2019 ES 201930405
(71) Applicant: Fertypharm, S.L., 08029 Barcelona (ES)
(72) Inventor: COSTILLAS PÉREZ, Roberto, 08029 BARCELONA (ES); DELPUEYO VERNIOL, José, Ángel, 08029 BARCELONA (ES)
(74) Representative: Herrero & Asociados, S.L.
(86) International application number: PCT/ES2020/070289
(87) International publication number: WO 2020/225468

(57) **Abstract**

The present invention refers to a food formulation characterized in that it comprises a mixture consisting of effective amounts of griffonia, saffron, hydrolyzed milk protein, magnesium and vitamin B6, the combination of which produces positive effects on the mood and vital state of an individual. Likewise, a food supplement is contemplated, comprising said formulation, in the form of a capsule for oral administration.

## Description

### Field of the invention

The field of application of the present invention is within the food sector. In particular, it relates to a combination of food ingredients that improves the mood and its use as a food supplement.

### Background of the invention

Food Supplements are products whose purpose is to supplement a normal diet and consist of concentrated sources of nutrients, or other substances, that have a nutritional or physiological effect, in a simple or combined way. They are marketed in dosed form, that is, capsules, pills, tablets, pills, which must be taken in small unit quantities.

They are not drugs, they improve the well-being of the person, but they do not cure any disease. They are intended for people in good health, and not ill people, so they do not need a prescription.

Among the great variety of marketed food supplements, we distinguish those intended to improve the mood and vital state of people.

In the state of the art, different nutrients or substances with positive effects on mood have been described. Thus, Griffonia simplicifolia, commonly known as Griffonia, is characterized by its high content of hydroxytryptophan (5-HTP), which is a natural amino acid and intermediate and precursor chemical compound in the biosynthesis of serotonin and melatonin, from tryptophan. Serotonin is a neurotransmitter involved in numerous functions such as promoting sleep, reducing anxiety, relieving the symptoms of depressive states and seasonal affective disorders, given its ability to improve mood, favoring the cognitive process, decreasing the sensation of hunger, etc. The therapeutic administration of 5-HTP has been shown to be effective in treating a wide variety of disorders including depression, fibromyalgia, anxiety associated with obesity, chronic headaches, and insomnia *(*Birdsall TC. 5-Hydroxytryptophan: A Clinically-Effective Serotonin Precursor. Alternative Medicine Review. 1998;3(4):10*).*

*Crocus sativus L.,* commonly known as Saffron, has been known since ancient times for its unique characteristics and chemical composition and has been considered a source of health, due to its proven qualities. Among all these qualities, it is worth focusing on two in particular: Its ability as an inhibitor of the enzyme monoamine oxidase (MAO), which leads to an increase in the concentration of neurotransmitters, improving mood and reducing anxiety, and its ability as a selective serotonin reuptake inhibitor, which leads to an increase in serotonin concentration, also improving mood *(*Lopresti y Drummond - 2014 - Saffron (Crocus sativus) for depression*;* De Monte et a/. - 2014 - New insights into the biological properties of Cro*;* Mazidi et. Al 2016; Boskabady y Farkhondeh - 2016 - Antiinflammatory, Antioxidant, and Immunomodulator*).*

In the state of the art, a milk protein hydrolysate containing a bioactive decapeptide with calming properties (lactium) is used. This peptide, also called α-Casozepine, is isolated from milk protein by "food grade" tryptic hydrolysis. This peptide reduces anxiety-related symptoms and helps consumers to better control their emotional state, whether induced by occasional or daily anxiety. Furthermore, it doesn't cause drowsiness, tolerance, memory loss, sedation, or addiction. It is completely natural and has no toxic effects on the body, even at high doses. GABA (gamma-aminobutyric acid) is a neurotransmitter, that is, an agent that transmits messages from one brain cell (neuron) to another. The message that GABA transmits is an inhibition message: it tells the neurons with which it comes into contact to slow down or stop transmitting. This translates into a general calming effect on the brain: in a way, it is the body's natural hypnotic and tranquilizer. α-Casozepine has a high affinity for the GABA receptor (the same receptor to which benzodiazepines bind) with which the activity of GABA is increased, giving rise to a calming effect that translates into a decrease in anxiety and a feeling of relaxation *(*Miclo L., Perrin E., Driou A., Papadopoulos V., Boujrad N., Vanderesse R., Boudier J.-F., Desor D., Linden D. & Gaillard J.-L. Characterization of α-casozepine, a tryptic peptide from bovine αS1-casein with benzodiazepine-like activity. FASEB J. 2001; 15(10):1780-2*)*

Magnesium is the fourth most abundant cation in the body and the second most important within the cell. It is involved in primitive biochemical processes such as cell adhesion; it acts as a regulator of the ribosome structure, in membrane transport, protein synthesis and nucleic acids. It acts in the generation and transmission of the nerve impulse, muscular and cardiac contraction, as well as in oxidative phosphorylation. Through numerous mechanisms, listed below, magnesium has been shown to help reduce tiredness and fatigue, as well as maintain normal psychological function.

Magnesium a) balances the entry/exit of calcium into the cell preventing it from being constantly excited, b) maintains the homeostasis of the Hypothalamic-Pituitary-Adrenal (HHA) axis, helping the release of cortisol and other stress hormones to stay within safe and healthy limits, c) acts as an antagonist of NMDA receptors (glutamate receptor, an excitatory neurotransmitter), helping to maintain glutamate excitability within a normal range, and d) Intervenes in the synthesis of neurotransmitters, so its presence is necessary for these synthesis processes to take place *(EFSA:* *http:*//*ec.europa.eu*/*food*/*safety*/*labelling nutrition*/ *claims*/*register*/*public*/*?event*=*getLatestVersionOfRegister;* Derom M-L, Sayón-Orea C, Martinez-Ortega JM, Martinez-Gonzalez MA. Magnesium and depression: a systematic review. Nutritional Neuroscience. 2013 Sep;16(5):191-206).

Vitamin B6 is a group of three chemical compounds: pyridoxine, pyridoxal, and pyridoxyamine. The phosphorylated derivatives of pyridoxal (pyridoxal phosphate - PLP) and pyridoxine (pyridoxamine phosphate - PMP) perform coenzyme functions. Pyridoxal phosphate is the metabolically active form of vitamin B6 and it acts as a coenzyme for many enzymes. It is involved in the metabolism of neurotransmitters that regulate mood, such as serotonin. It is responsible for the conversion of 5-HTP into serotonin and this in turn can be transformed into melatonin. In addition, it is involved in the synthesis of dopamine, adrenaline, norepinephrine and GABA. In short, the presence of vitamin B6 in the body is necessary for the synthesis of neurotransmitters to occur and these lead to general well-being, improving mood, reducing anxiety and regulating the sleep cycle. *(*Kennedy D. B Vitamins and the Brain: Mechanisms, Dose and Efficacy-A Review. Nutrients. 2016 Jan 27;8(2):68*.* Birdsall TC. 5-Hydroxytryptophan: A Clinically-Effective Serotonin Precursor. Alternative Medicine Review. 1998;3(4):10*).*

The authors of the present invention, after significant experimental work, have developed a new food formulation, in the form of a food supplement, that improves the mood by increasing neurotransmitters (serotonin, dopamine, noredrenaline, GABA) and decreasing the cortisol. It is the first food supplement specially formulated for low vitality.

The formulation comprises a mixture consisting of effective amounts of griffonia, saffron, hydrolyzed milk protein, magnesium, and vitamin B6. The synergies established between its ingredients, at specific concentrations, allow maintaining a positive mood.

In addition, the new formulation does not have interactions with fertility treatments, and is also suitable for diabetics, vegans, lactose and gluten intolerant people. This is due to the use of natural, high-quality, safe and well-tolerated ingredients:
α -Casozepine is a component with mutagenicity, teratogenicity and toxicity studies carried out in animals, wherein it is observed that there are no cytotoxic or teratogenic effects. These toxicity studies have been conducted throughout pregnancy in experimental animals compared to placebo. No induction of internal or external malformations is observed either.

Ingestion of α-Casozepine does not alter the duration of pregnancy, maternal behavior or the brood care . Nor does it induce problems in physical, neuromotor, behavioral and cognitive development in the offspring.

Regarding saffron, studies have shown that the supplementation of in vitro culture media with 10 µg/ml of crocin (Crocus Sativus L.) significantly increases nuclear maturation and preimplantation development, compared to the control group.

Griffonia is a serotonin precursor that helps to keep proper serotonin levels to maintain a positive mood. It does not present special interactions, since it becomes an essential neurotransmitter.

Vitamin B6 helps to convert 5-HTP into serotonin. Vitamin B6 is safe during pregnancy and breastfeeding when used orally in doses that do not exceed the recommended daily allowance (RDA).

Magnesium is an effective and safe supplement, it has multiple EFSA approvals: it contributes to the reduction of tiredness and fatigue, contributes to the normal functioning of the central nervous system, contributes to a normal psychological function, etc. It is a safe product for women seeking pregnancy or being pregnant.

### Description of the invention

The authors of the present invention have developed a new food formulation to improve the mood and vital state of an individual.

Thus, in a main embodiment of the invention, a new food formulation is contemplated that comprises a mixture of food ingredients consisting of effective amounts of griffonia, saffron, hydrolyzed milk protein, magnesium and vitamin B6, the combination of which produces positive effects on the mood and vital state of an individual.

Griffonia is a medicinal plant whose seeds are used for their high content of 5-hydroxy-1-tryptophan (5-HTP), a direct precursor of serotonin synthesis. 5HTP, as the main active ingredient, acts by promoting serotonin synthesis, reducing stress and the risk of depression, mainly by generating an increase in serotonin production. In particular embodiments of the new formulation, griffonia is presented in the form of a 98% griffonia extract.

Saffron contains four active ingredients: crocin, crocetin, picrocrocin and saffranal.

The main active ingredient in saffron is saffranal. In particular embodiments of the formulation of the invention, the saffron used in the formulation of the invention is presented in the form of saffron extract, with 2% saffranal. This ingredient has several beneficial effects, and its antidepressant and anxiolytic effect, as well as an improvement in learning, has been shown. Its main mechanism of action is by inhibiting the reuptake of serotonin, norepinephrine and dopamine, providing a very marked antidepressant and serotonergic effect.

In particular embodiments of the formulation of the invention, the hydrolyzed milk protein used is a hydrolyzed αS1-casein tryptic containing α-Casozepine as the main active ingredient. This peptide has a high affinity for the benzodiazepine binding site, the central GABAA receptor. The binding of this peptide with the GABBA receptor demonstrates anxiolytic and antidepressant properties and reduces stress.

Magnesium, in particular embodiments of the formulation of the invention, is added to the formulation in the form of heavy magnesium oxide. Magnesium has an antidepressant effect. Its deficiency causes an increased risk of depression and anxiety.

Finally, in particular embodiments of the formulation of the invention, the vitamin B6 included in the formulation is pyridoxine hydrochloride. This ingredient has a concomitant effect with 5-HTP to convert to serotonin.

Thus, in a preferred embodiment of the invention, the formulation of the invention comprises 98% griffonia extract, 2% saffron extract, α-casezepine, heavy magnesium oxide and pyridoxine hydrochloride.

In another main aspect of the invention, the use of the new formulation in the preparation of a food supplement with positive effects on the mood and vital state of an individual is contemplated.

This new combination of ingredients, developed by the authors of the invention, does not present interactions with fertility treatments, since it is composed of natural products such as saffron, magnesium, vitamin B6, α-Casozepine and griffonia. These ingredients are commonly found in foods and have a beneficial function on mood and vitality. Its main function is to improve neurotransmitter levels and reinforce the role of a natural diet. In the case of griffonia, it is a precursor of 5-HT to increase serotonin levels. The intake of foods such as oily fish, meat, eggs, cereals, etc., favors the increase of tryptophan, which is converted in the body into 5 HTP. Griffonia is a natural source of 5HTP, exercising the same function as the intake of these foods. Therefore, the formula presented allows maintaining a positive mood during the fertility processes. In addition, the safety and quality of its ingredients makes the food supplement suitable for diabetics, vegans, lactose and gluten intolerant people

Another main embodiment of the invention relates to the food supplement comprising the formulation of the invention.

In preferred embodiments of the invention, the food supplement is formulated to be administered orally, preferably in capsule form, although it can also be formulated in tablet or powder format for oral dissolution.

Thus, in another main aspect of the invention a capsule comprising the food supplement of the invention is contemplated. In preferred embodiments, the capsule is formulated based on the following composition by weight:

| Active ingredient | Amount per capsule (mg) |
|---|---|
| Griffonia Extract 98% 5-HTP | 25.50-50 |
| Heavy magnesium oxide | 100-200 |
| Saffron extract 2% | 14-28 |
| Milk protein hydrolysate | 75-150 |
| Pyridoxine Hydrochloride B6 | 0.85-1.70 |

The proposed dose range allows the individual to be administered one or two capsules a day, the most frequent dose being that of two capsules a day, and the one with the greatest effect on mood.

In a preferred embodiment, the amount of each ingredient (mg) per capsule is as follows:

| Active ingredient | Amount per capsule (mg) |
|---|---|
| Griffonia Extract 98% 5-HTP | 25,50 |
| Heavy magnesium oxide | 100,00 |
| Saffron extract 2% | 14,00 |
| Milk protein hydrolysate | 75,00 |
| Pyridoxine Hydrochloride B6 | 0,85 |

Likewise, the addition of different excipients necessary for the formulation of the capsule is contemplated, such as, for example, calcium phosphate, magnesium stearate or silica.

Below, we provide by way of example, without being considered limiting or restrictive of the present invention, the following formulation obtained:

### Example

| INGREDIENTS | mg/doses | Function (*) |
|---|---|---|
| Emdex | 146.836 | Ingredient |
| Magnesium oxide (59.7%) high density 1/1 (G/CG) | 100.503 | Active ingredient |
| Lactium milk protein hydrolysate | 75.000 | Ingredient |
| Griffonia seed E.S. > 98% 5-HTP | 25.511 | Active ingredient |
| Tribasic calcium phosphate (E-341) | 15.600 | Antiaggregate |
| Saffron extract> 2% saffranal | 14.000 | Active ingredient |
| Vegetable magnesium stearate (E-470b) | 7.800 | Antiaggregate |
| Precipitated Silica, Ibersil D250 (46.7% Si) (E-551) | 3.900 | Antiaggregate |
| Vitamin B6 (Pyridoxine HCI 82.7%) CG | 0.850 | Active ingredient |
| TOTAL WEIGHT CONTENT | 390.000 | |
| Jelly | 94.080 | Ingredient |
| Titanium Dioxide (E-171) | 1.920 | Dye |
| White capsule "0" (44,000) | 96.000 | |
| Capsule total weight | 486.000 | |

The recommended daily allowance for this particular formula is 2 capsules.

## Claims

1. Food formulation **characterized in that** it comprises a mixture consisting of effective amounts of griffonia, saffron, hydrolyzed milk protein, magnesium and vitamin B6, the combination of which produces positive effects on the mood and vital state of an individual.

2. Food formulation according to claim 1, **characterized in that** it comprises 98% griffonia extract, 2% saffron extract, α-casezepine, heavy magnesium oxide and pyridoxine hydrochloride.

3. Use of a food formulation according to claim 1 or 2 in the preparation of a food supplement with positive effects on the mood and vital state of an individual.

4. Food supplement comprising a food formulation according to claim 1 or 2.

5. Food supplement according to claim 4 formulated for oral administration.

6. Food supplement according to claim 5 formulated in capsule form.

7. Capsule comprising a food supplement according to any of claims 4-6.

8. Capsule, according to claim 7, which is formulated according to the following concentration ranges of each ingredient:
| Active ingredient | Amount per capsule (mg) | |
|---|---|---|
| Griffonia Extract 98% 5-HTP | | 25-50 |
| Heavy magnesium oxide | | 100-200 |
| Saffron extract 2% | | 14-28 |
| α-casezepine | | 75-150 |
| Pyridoxine Hydrochloride | | 0.85-1.70 |

9. Capsule, according to claim 8, which is formulated according to the following amounts of each ingredient:
| Active ingredient | Amount per capsule (mg) |
|---|---|
| Griffonia Extract 98% 5-HTP | 25.50 |
| Heavy magnesium oxide | 100.00 |
| Saffron extract 2% | 14.00 |
| α-casezepine | 75.00 |
| Pyridoxine Hydrochloride | 0.85 |
